# EUROPEAN PATENT APPLICATION

(11) **EP 4 151 784 A1**
(43) Date of publication of application: **22.03.2023**
(21) Application number: 21804101.0
(22) Date of filing: 14.05.2021
(51) Int. Cl.: C40B 30/04, C12N 15/10, G01N 33/543

(54) **RAPID SINGLE-STEP GRADIENT METHOD FOR THE GENERATION OF NANOANTIBODIES**

(30) Priority: 15.05.2020 US 202063025534 P
(71) Applicant: Universidad Austral de Chile, Valdivia, 5110566 (CL)
(72) Inventor: ROJAS, Alejandro, Valdivia, 5090000 (CL); VALENZUELA, Guillermo, Valdivia, 5090000 (CL); JARA, Ronald, Valdivia, 5090000 (CL); HIMELREICHS, Johanna, Valdivia, 5090000 (CL); SALINAS, Constanza, Valdivia, 5090000 (CL); PINTO, Teresa, Valdivia, 5090000 (CL); LÓPEZ, Natalia, 28938 Madrid (ES); CHEUQUEMILLA, Yorka, Valdivia, 5090000 (CL); CUEVAS, Alexei, Valdivia, 5090000 (CL); MIRANDA, Zaray, Montes de Oca San Jose, 11501-2060 (CR); UBERTI, Benjamín, Valdivia, 5090000 (CL); MULLER, Ananda, Basseterre, 60173 (KN)
(74) Representative: Elzaburu S.L.P.
(86) International application number: PCT/CL2021/050039
(87) International publication number: WO 2021/226728

(57) **Abstract**

One-step rapid gradient method for rapid generation of nanoantibodies (HHV) against any antigen. Where the method comprises the separation of nanoantibodies against a specific antigen from a nanoantibody expression library through the following steps:
(a) binding the antigen of interest to beads made of protein-binding polymers
(b) incubating the microorganisms of the expression library with the pearls of stage (a),
(c) placing the beads of step (b) in a tube with an inert medium with a density greater than or equal to 1 g/mL and centrifuge for 45 s to 2 minutes at a rate between 150 and 250 g;
d) discarding the upper fraction and supernatant with the free microorganisms, and obtain the beads with the linked microorganisms, which correspond to those that express HHV that recognizes the antigen.

## Description

### Field of invention

The invention relates to a new one-step fast gradient method for the rapid separation of nanoantibodies against any antigen.

### State of the art

Some naturally occurring antibodies lack light chains, known as single-domain antibodies (HCAb). They are derived from IgG and are found throughout the family Camelidae. The Camelidae or camelid family is composed of camels, dromedaries, llamas, vicuñas, guanacos and alpacas. The antigen-binding fragment of an HCAb contains a single variable HHV domain consisting of 3 hypervariable regions (RDA). When isolated, the HHV domain is also known as a nanoantibody. Target-specific nanoantibodies derived from camelid HCAbs are obtained after immunization with the antigen, plus the adjuvant. Our platform has developed an improved procedure for obtaining nanoantibodies by using alpacas as a donor species.

To isolate the genetic sequences of target-specific nanoantibodies produced after immunization, we must first isolate peripheral B cells to obtain the total RNA, followed by cDNA preparation to finally amplify the nanoantibody region. The cDNA fragment encoding the nanoantibody is as short as 360 nt, and up to ~3 × 10⁶ individual clones can be obtained from 120 ml of blood from an immunized alpaca. A bacteria or yeast visualization system is used to clone the various complete individual nanoantibodies, generating what is known as a nanoantibody or HHV library.

Microorganism presentation technology allows nanoantibodies to be expressed on the surfaces of microorganisms and therefore researchers can separate and enrich bacteria or yeasts expressing the nanoantibody of interest on their surface by means of affinity purification. Final isolated nanoantibodies are expressed recombinantly in bacteria and their binding capabilities can be characterized by ELISA and quantitative biochemical parameters such as ITC. In addition to targeted immunization, it is also possible to use very large libraries of nanoantibodies (1×10⁹) to find binding nanoantibodies using a stochastic approach. The nanoantibodies are then produced in a renewable and economical manner.

In fact, the inventors demonstrated that specific nanoantibodies against a particular antigen from a bacterial presentation library can be selected with the protocol of the invention in a quick and economical manner by the use of common reagents and a conventional centrifuge. This procedure may also be applicable to yeast presentation libraries.

Today, more than at any time in modern history, diagnostic and neutralization measures are urgently needed to control a global pandemic. Recombinant antibodies, including alpaca nanoantibodies, are excellent candidates for this challenge. However, the process of producing a classic nanoantibody takes months, for example, complete immunization takes an average of 8 weeks, followed by building the nanoantibody library and selecting affinity of clones, which takes about 6 months even through the use of advanced and combined sequencing and mass spectrometry techniques (Pardon, Els, et al. "A general protocol for the generation of Nanobodies for structural biology." Nature protocols 9.3 (2014): 674-693) or classical phage presentation techniques (Verheesen, P., & Laeremans, T. (2012). Selection by phage display of single domain antibodies specific to antigens in their native conformation. In Single Domain Antibodies (pp. 81-104). Humana Press, Totowa, NJ.).

Here, we optimize a simple, fast, and inexpensive density gradient method for selecting nanoantibodies that bind to the antigen of interest.

### Nanoantibodies

In nature, there are some exceptions to functional antibodies that lack light chains, known as single-domain antibodies (HCAb). They are derived from true IgG2 and IgG3 type IgG and are found throughout the Camilidae family, and also in nurse sharks, orectolobids and perhaps spotted ratfish. The Camilidae or camelid family is composed of camels, dromedaries, llamas, vicuñas, guanacos and alpacas. The antigen-binding fragment of an HCAb contains a single variable HHV domain consisting of 3 hypervariable regions. When isolated, the HHV domain is also known as a nanoantibody. Target-specific nanoantibodies derived from camelid HCAb are usually obtained rapidly after immunization with the most adjuvant target protein. Analysis of the structure of the nanoantibody reveals how hypervariable regions project in loops outside the structure of the nucleus.

Among the advantages of nanoantibodies we find their small size, they can be humanized, their structure and behavior are stable in aqueous solutions, their specific binding and high affinity to a single target protein and their natural production by camelids. Therefore, nanoantibodies are the best tools available today for affinity-based diagnostics and therapies.

Here, the advantages and uses of nanoantibodies are summarized in detail.

### Advantages of nanoantibodies

### Purification

Purification of nanoantibodies is simple compared to any other source of antibodies. They are often expressed attached to an affinity label, such as 6× histidine labels, to allow affinity purification. Enrichment often sets in the bacterial periplasm where the oxidizing environment allows for the formation of suitable disulfide bonds. Several milligrams of a liter of culture can be isolated and recombinant isolated nanoantibodies can be further isolated using standard biochemical techniques.

### Stability

Nanoantibodies are small, compact polypeptides and are often expressed in the periplasm of bacteria. They are very stable at high temperatures, starting at 6 °C compared to human VH, and are also resistant to denaturing chemical agents. In addition, molecular engineering of the nanoantibody structure has shown that stability increases when a cysteine is introduced at positions 54 and 78 to form an additional disulfide bond. Interestingly, the resulting superstable nanoantibody is also more resistant to proteases such as pepsin or chymotrypsin.

### Immunological invisibility

Nanoantibodies can be used as therapeutic bullets against tumors, pathogens and chronic diseases, however, as foreign proteins, they could trigger an immune response on their own. Fortunately, the small size, rapid blood clearance and high homology with the human variable region of the VH heavy chain make them immunogenically small. Only some amino acids differ between nanoantibodies and human OAB, replacing these camelid amino acids with human amino acids has been used to humanize camelid nanoantibodies and make them even safer for therapies.

### Accessibility

Nanoantibodies are strict monomers, their affinity for substrates depends on the projection of the three hypervariable loops. Consequently, nanoantibodies tend to interact with cavities of the spatial structure of polypeptides, but not efficiently with peptides. For example, several identified nanoantibodies directly block active enzyme sites. The FC5 nanoantibody can even cross the blood-brain barrier via transcytosis and form partially bispecific antibodies used for therapeutic approaches. ^{52.53.} Finally, molecular accessibility impacts access to macromolecular complexes.

### Use of nanoantibodies

### Cell biology and molecular biology tools

After genome sequencing, new fields began to gain territory: the proteome and the interactome. The interacting partners of a protein define several of its regulatory functions and mechanisms. Today, modern tools based on proteomic approaches can accurately identify interactors even when they are in low concentrations. However, the limiting step is how to selectively enrich a particular protein and its interactants. Nanoantibodies have been used for the purification by affinity of proteins in a specific way and even for the detection of post-translational modifications. However, the number of nanoantibodies available is very limited and many more are needed. Nanoantibodies can be used for various experimental environments such as ELISA, immunoprecipitation, CHIP-seq, immunostaining, to induce degradation of specific proteins by recruiting degradation machinery and blocking and activating cellular pathways. A nanoantibody obtained from alpacas specifically recognizes the green fluorescence protein, known as the GFP trap system, became the first-line tool for interaction and proteomics studies based on its extraordinary affinity and ability to bind to GFP fusion proteins. Today, it can even be used for GFP extraction from endogenously labeled proteins in combination with CRISPR/Cas9. GFP nanoantibody can also be coupled to fluorescent dyes to detect GFP by microscopy approaches when GFP levels are very low.

### Structural studies

When a nanoantibody binds to a three-dimensional structure, some properties of the target protein change. One of the common features is that nanoantibodies limit the conformational changes of the target protein by promoting a unique conformation step. Surprisingly, these phenomena positively impact the possibilities of crystallization, therefore, nanoantibodies have been used as helpers to facilitate protein crystallization.

### Diagnostics

Nanoantibodies are a superior tool for diagnosis. Their unlimited *in vitro* production capacity makes nanoantibodies more reliable than conventional antibodies and independent of batch preparation or animal serum limitations. Nanoantibodies can be produced as a protein fused with informant peptides or proteins for staining or direct visualization, including affinity tags (Flag, HA, V5, and cMyc), fluorescent proteins (GFP, RFP, etc.), and enzymes for colorimetric measurements such as horseradish peroxidase (HRP). For example, ELISA assays can be improved by the use of nanoantibodies for specific immobilization or detection by the use of a specific nanoantibody coupled to horseradish peroxidase (HRP).

Nanoantibodies meet most of the requirements of an ideal probe for successful molecular imaging. Several imaging techniques such as SPECT, PET, optical imaging and ultrasound have been successfully employed for molecular imaging by the use of in vivo nanoantibodies. In SPECT, specific targeted nanoantibodies are coupled to an irradiation source γ administered systemically. The nanoantibodies are then detected in a whole-body scanner. The PET strategy is similar to SPECT, but instead uses positron-emitting radionuclide-labeled nanoantibodies. These imaging technologies are used for various tumor diagnostic techniques in animal models: for example, an anti-EGFR nanoantibody can be used to detect human squamous cell carcinoma and human prostate carcinoma. In another extraordinary case, they have used a combination of nanoantibodies targeting carbonic anhydrase IX (CAIX) and human epidermal growth factor receptor 2 (HER2) proteins conjugated with different fluorophores. Here, the authors conclude that simultaneous detection of the expression status of multiple clinically relevant tumor markers could lead to better detection of primary tumors and their metastases.

Today, the best chances of cancer survival are early detection and timely surgery. Thus, *in vivo* detection based on nanoantibodies is one of the most promising future technologies to fight cancer.

### Therapies

Several nanoantibodies have been developed in the context of different experimental therapeutic applications against different viruses: HIV, hepatitis B virus, influenza virus, respiratory syncytial virus, rabies virus, foot-and-mouth disease virus, poliovirus, rotavirus and PERV. Surprisingly, nanoantibodies can neutralize HIV infection; cell-to-cell spread has been inhibited by the use of HIV isolated from patients. Due to the low immunoreactivity of nanoantibodies, they can be injected into patients with very few or no side effects. To make them more efficient and specific, nanoantibodies can be linked to produce bivalent, multivalent, and/or multispecific nanoantibodies, or combined with other nanoantibodies or circulating proteins such as albumin to increase their turnover and therapeutic effectiveness. The rabies virus causes a lethal brain infection in people. Shortly after exposure, rabies prophylaxis with immunoglobulins and plasma-derived vaccines is administered. Often, this occurs directly after the attack of an animal that might be infected. Anti-rabies nanoantibodies can significantly prolong survival or even completely cure the disease in animal models. The respiratory syncytial virus, RSV, is one of the leading causes of hospitalization in children, each year more than 1.9 million children under 1 year of age are infected and there are more than 0.3 million children under 5 years of age hospitalized. Current RSV therapy is not available. However, trivalent nanoantibody-based therapy is in phase II clinical trials. The absolute novelty of the RSV therapy developed by Ablynx, ALX-0171, is the direct neutralization of the virus in the lung of infected experimental animals. The nanoantibody is administered by nebulization and reduces the virus titer by 10,000 times. Nanoantibodies are also used for cancer immunotherapies.

According to the above, a method such as the invention that allows for rapid selection of HHV with affinity to an antigen of interest has a great application in the industry for generation of these nanoantibodies for all the uses already described.

### Description of the figures.

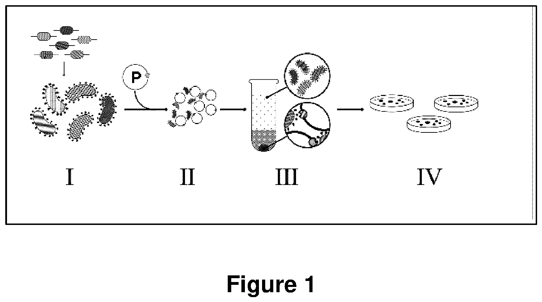

**Figure 1****.** Schematic representation of the protocol for HHV isolation using density gradient separation: the bacterial presentation library (I) expressing HHV on the surface of bacteria is briefly incubated (II) with conventional sepharosase beads coated with the antigen of interest. Immediately after depositing the mixture in a conical Ficoll density gradient (III) tube and centrifuging at 200 g for 1 min, the beads pass through the sequential selection of the density gradient to the bottom of the tube with the bacteria expressing specific HHV, while the unbound bacteria remain on the gradient surface. Next, the beads are resuspended and (IV) the incubating bacterial clones are isolated in agar plate.

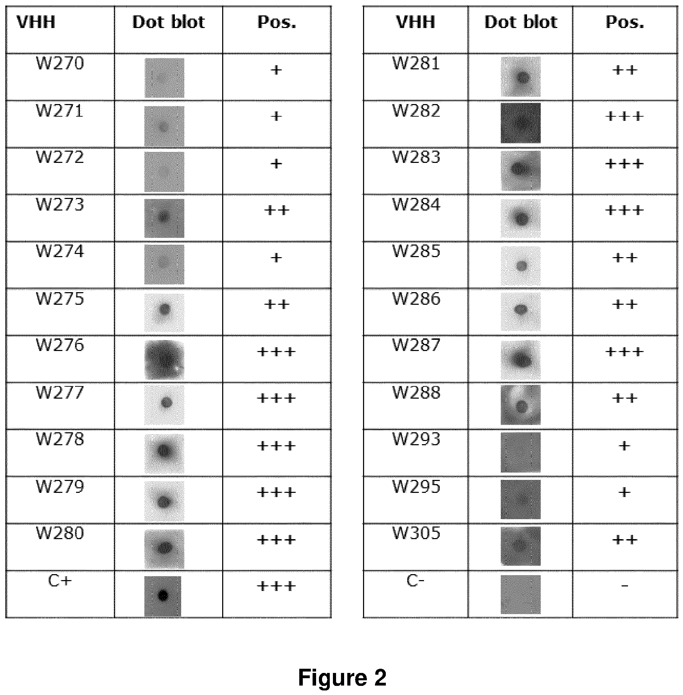

**Figure 2** Immunodetection of Spike-SARS-CoV2 in a nitrocellulose membrane with a pore size of 0.2 µm with the HHV of the invention as primary antibody, followed by mouse anti-Myc and goat anti-mouse IgG coupled to HRP. It includes photograph of each reaction for different nanoantibody selected with the method of the invention, as well as qualitative assessment (+, ++, +++)

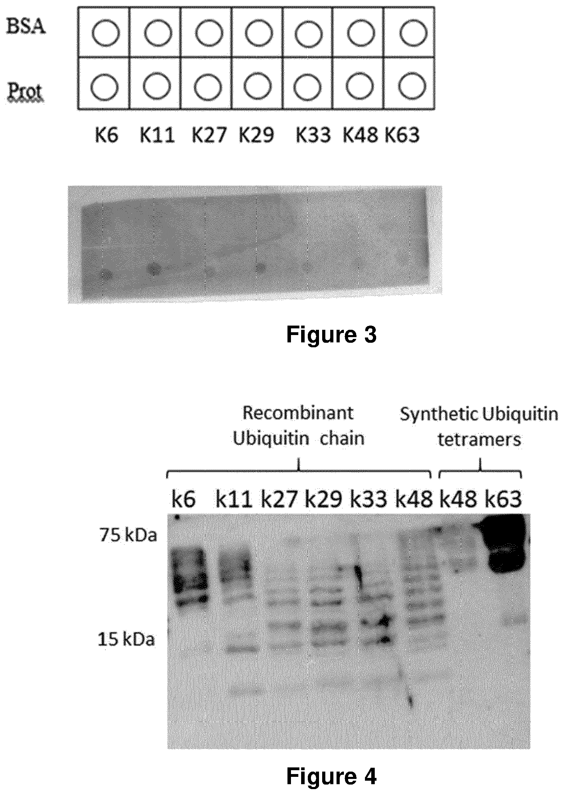

**Figure 3** Dot Blot against recombinant and synthetic polyubiquitin chains, by the use of Nb No. 34, mouse anti-myc and HRP-coupled anti-mouse. Nb No. 34 was expressed in the plasmid pNae2 (fused with intimin) with labels 6×His and Myc.
**Figure 4** Western Blot against recombinant and synthetic polyubiquitin chains, by the use of Nb No.34, mouse anti-myc and mouse anti-HRP. Nb No. 34 was expressed in BL21 cells by the use of pNae2 (without intimin) with the Nb coding sequence between 6×His and Myc.

### Detailed description of the invention

The invention allows for obtaining nanoantibodies quickly and easily, against any type of antigen, such as viruses, bacteria or microorganisms usually proteins or molecules, among others. But due to its speed, the method of this invention is very efficient for the generation of nanoantibodies against pathogens of emerging diseases, such as COVID.

The invention relates to a method of separation of nanoantibodies (HHV) against a specific antigen from a nanoantibody expression library, wherein the method comprises the following steps:
(a) binding the antigen of interest to polymeric beads that allow protein binding
(b) incubating the microorganisms of the expression library with the beads of step (a), where the expression library corresponds to microorganisms transformed with the cDNA of fragments corresponding to the HHV domains of HHV-producing animal previously immunized with that antigen of interest, or synthetic libraries;
(c) placing the beads of step (b) in a tube with an inert medium with a density greater than or equal to 1 g/mL and centrifuging for 45 s to 2 minutes at a rate between 150 and 250 g;
d) discarding the upper fraction and supernatant with the free microorganisms, and obtaining the beads with the linked microorganisms, which correspond to those that express HHV that recognizes the antigen.

Where the beads used are sepharose, cellulose, latex, agarose or nickel and can be modified to bind proteins, and have a density greater than the medium used in (c). These beads bind to the antigen of interest in a stable manner in a time of between 2 to 12 hours and then the sites that have not reacted with the antigen are blocked, by any means available in the technique.

The bond between the beads and the antigen can be non-covalent covalent.

Where this inert medium of stage (c) is preferably chosen between Ficol, percol or sucrose.

To ensure that the microorganisms in the expression library are expressing HHV they should be treated with a protein expression inducer for 2 to 4 hours prior to step (b). In one embodiment, the protein expression inducer is IPTG at a concentration between 20 to 100 µM. Later in step (b) the microorganisms of the expression library are incubated with the beads attached to the antigen for between 20 to 60 minutes at room temperature.

Finally, after separation in the density gradient, in step (d) the beads are washed with PBS and seeded on agar plates with culture medium in order to obtain isolated colonies. Where each colony corresponds to a microorganism that expresses an HHV capable of binding to the antigen of interest.

First, you must have a nanoantibody or HHV library.

If you want to generate this library yourself, you require a purified antigen, which can be obtained with a system of expression of baculovirus, bacteria, yeast or directly from its natural source. Next, an animal producing nanoantibodies, such as an alpaca or llama, is immunized between one- to four times with 50 to 500 µg of the antigen. The immune response of alpaca serum against the antigen after immunization can be observed rapidly and qualitatively by means of Dot Blot analysis, by immobilization of the epitope to a nitrocellulose membrane and by the use of alpaca serum as a source of primary antibodies; or analytically and comparatively by means of ELISA by the use of the complete antigen that is immobilized in the ELISA plate and the alpaca serum as a source of primary antibodies. Thus, a bacterial presentation library of individual nanoantibody clones is quickly built.

Once the library is obtained, the method of the invention is applied for the selection of nanoantibodies based on a density gradient, for example the simple use of Ficoll, percol or sucrose. Ficoll is an inexpensive reagent available worldwide commonly used for the fragmentation of blood samples. The inventors developed the method of the invention inspired by the main observation that red blood cells accumulate at the bottom of the Ficoll density gradient, while PBMCs remain in the upper fraction.

The inventors created a method using beads made of polymers such as conventional sepharose or agarose which, in a density gradient, such as one obtained by the Ficoll reagent have shown that the density of the chosen beads was suitable for precipitating at the bottom of a 15 ml tube. At the same time, the inventors showed that, when performing the same test with free bacteria, the bacteria remained in the upper fraction. Revealing the possibility of separating free bacteria from those that express HHV and bind beads with the antigen, in a density gradient.

To obtain specific clones of HHV or nanoantibodies, a bacterial presentation system is usually used, where each bacterium expresses a single type of nanoantibody in its outer bacterial membrane after IPTG induction. Therefore, the inventors obtained sefarose beads coated with the antigen of interest to bind to individual bacteria expressing specific nanoantibodies on their surface against said antigen. Thus, bacteria expressing an HHV or nanoantibody that binds to the antigen sink to the bottom of the density gradient, while unbound bacteria would remain in the upper fraction (Figure 1).

### Examples

### Example 1. The simple density gradient method of the invention and its use to obtain nanoantibodies against the Spike protein of SARS-CoV-2

First, the inventors obtained the lyophilized SARS-CoV-2 Spike protein generated in a baculovirus expression system. Prior to immunization, Spike protein integrity was tested by SDS-Page and Coomassie staining (Figure 1a). An alpaca named Buddha (Figure 1b) was immunized twice with 100 µg of the entire Spike protein. The immune response of alpaca serum prior to immunization revealed a fortunate basal cross-reaction against the Spike protein. Then, after the second immunization, a significant increase in IgG antibodies was observed in alpaca serum qualitatively rapidly by Dot Blot analysis, by immobilization of the epitope in a nitrocellulose membrane and by the use of alpaca serum as a primary antibody source (Figure 1c). In addition, the inventors verified the increase of IgG antibodies (analytically and comparatively) by means of ELISA by the use of the complete Spike protein immobilized in the ELISA plate and by the use of alpaca serum as a source of primary antibodies (Figure 1d). Therefore, the inventors quickly built a bacteria presentation library consisting of 2.3 × 10⁶ clones of individual nanoantibodies with 0.7% vector religation.

### Building immunization and HHV libraries

The alpaca immunization process followed the protocol "Animal use in research" generated by the Bioethics Committee of the Universidad Austral de Chile. One day before immunization, 5 ml of blood was collected for preimmune serum testing. For immunization (day 1), 100 µg of full-length SARS-CoV2 spike protein (SINOBiological) was used. The cold lyophilized protein was dissolved in 2 ml of adjuvant (veterinary vaccine adjuvant, GERBU FAMA) diluted 1:1 in sterile water and injected subcutaneously into a male alpaca (*Vicugna pacos*)*.* A total volume of 4 ml was injected into four different sites of the alpaca. A 5 ml blood sample was collected seven days after the first immunization. On day 14, the alpaca was immunized again with 100 µg of Spike and on day 15 a 120 ml sample of blood was collected from the jugular vein in tubes containing 3.8% sodium citrate as an anticoagulant. The noncoagulated blood sample was mixed with the same volume of calcium-free HBSS medium (Gibco), divided into 10 ml aliquots, and 5 ml of Ficoll-Paque Premium (GE Healthcare) was added on top of each aliquot in 15 ml of sterile Falcon tubes. After centrifugation (1,200 x rpm, 80 min, room temperature), the PBMC fraction was recovered from the interface, washed twice in HBSS by centrifugation (3,500 x rpm, 10 min), resuspended in 4 ml sterile PBS 1X (Gibco). RNA extraction and cDNA production were carried out using the commercial RNeasy Mini (Qiagen) kit and the QuantiTect Reverse Transcription Kit (Qiagen), respectively. Approximately 2 µl of each synthesized cDNA was used as a template in a total PCR reaction volume of 50 µl with the oligonucleotides CALL001 (5'-GTC CTG GCT CTC TTC TAC AAG G-3') and CALL002 (5'-GGTACGTGCTGTTGAACTGTTCC- 3') (Conrath *et al.,* 2001). The amplified fragments of ~0.6 kb, corresponding to the VHH-CH2 domains, and ~0.9 kb, corresponding to the conventional VH-CH1-CH2 domains, were separated into 1.2% low melting point agarose gel (w/v) and a band of ~0.6 kb was purified (QIAEX II Gel Extraction Kit, Qiagen). This fragment was used as a template in a second PCR reaction with oligonucleotides VHH-Sfi2 (5'-GTC CTC GCA ACT GCG GCC CAG CCGGCC ATG GCT CAG GTG CAG CTG GTG GA-3') and VHH-Not2 (5'- GGA CTA GTG CGG CCG CTG AGG AGA CGG TGA CCT GGG) T-3') to finally obtain the amplified fragments of ~0.4 kb, corresponding to VHH domains. The amplified HHV fragments were digested with *Sfi* I and *Not*I (Thermo Scientific) restriction enzymes and bound at the same sites of the purified pNeae2 vector (Salema *et al.,* 2016). The ligations were subjected to electroporation in *cells of E. coli* DH10B-T1 R achieving a library size of 2.3 × 10⁶ individual clones, as determined by seeding on LB-Cloramphenicol agar plates with glucose at 2% w/v incubated at 30 °C. Less than 0.7% of vectors re-linked from a control ligation carried out in parallel without the DNA insert was estimated. The transformed bacteria were scraped off the plates and stored at -80 degrees in LB broth with 30% glycerol.

Once the library was obtained, the inventors applied the method of the invention for the selection of nanoantibodies based on a simple density gradient by the use of Ficoll. (Figure 1).

### Coupling epitopes to beads

1 ml of NHS-activated sepharose 4 Fast Flow beads (General Electric) with 2 ml of cold HCl 1 mM were washed immediately before use, then washed 5 times with PBS (Saline phosphate buffer) 1Xsterile cold. 200 µg of purified protein in PBS 1X were added to the beads and incubated in rotation until the next day. The groups that did not react in the medium were blocked by the addition of ethanolamine at a final concentration of 0.5 M. The beads were washed 5 times with PBS 1X and stored at 4°C.

### Density gradient separation

1 ml of glycerol stock solution from the library was inoculated into a flask containing 20 ml of LB medium with 25 µg/ml chloramphenicol and 2% glucose. The flask was incubated (pre-inoculum) until the next day at 37 °C with 200 rpm stirring. The same procedure was repeated with control bacteria transformed with a kanamycin-resistant plasmid (control). The preinoculum was sedimented and resuspended in LB medium with 25 µg/mL chloramphenicol and then diluted to 0.02 optical density at 600 nm in 100 ml of fresh LB medium with 25 µg/mL chloramphenicol without glucose, incubated at 37 °C with 200 rpm stirring to reach an optical density of 600 nm of 0.45 to 0.6. IPTG was added at a final concentration of 50 µM to induce protein expression for 3 hours at 30 °C and 200 rpm. The OD 600 nm absorbance of the library and control bacteria cultures was measured. 50 mL of both cultures were washed three times with 10 mL of PBS 1X filtered. Centrifugation was always at 3000 x g for 5 min. Both cultures were resuspended in a final volume of 10 ml of PBS 1X. 2 ml of library culture and 2 ml of control culture were mixed (if the final 600 nm optical density was the same, if not, the volume of the control bacteria was adjusted according to the OD to ensure an equal number of bacteria) and incubated with 300 µL of beads coupled to the epitope in a 15 ml conical tube on a rotating platform for 30 min at temperature environment. The mixture was added to Ficoll 6 ml (Ficoll-PaqueTM PLUS GE Healthcare) in a 15 ml conical tube and centrifuged at 200xg for 1 min. The unbound fraction was discarded (upper fractions).

The visible bead sediment contains bacteria that express an HHV that binds to antigens, in this case Sars-CoV2 Spike. This sediment was resuspended in 4 ml of PBS 1X and rotated for 5 min at room temperature. This step was repeated six times, in order to eliminate any bacteria not attached to the beads.

In order to amplify the bacteria attached to the beads, 1 mL of LB medium was added and incubated for 5 min at room temperature, then 50 µL were seeded in LB agar plates with 25 µg/mL chloramphenicol and 2% glucose a, incubated at 37 °C until the next day (> 20 hours recommended).

Where each colony obtained corresponds to a bacterium containing a nanoantibody or HHV that binds specifically to the antigen of interest.

### Binding test of selected HHV to SARS-CoV-2 spike

To verify that the nanoantibodies of the invention bind to the Spike protein, an immunoblot was made for the 22 nanoantibodies, which faced Spike-GFP expressed in natural viral conditions in human cells (Figure 2).

It was observed that all nanoantibodies selected by the method of the invention were attached to the Spike antigen of SARS-CoV-2. The results demonstrate that the method of the invention allows to quickly and efficiently select nanoantibodies that bind specifically to an antigen of interest, such as the SARS-CoV2 Spike.

### Ubiquitin

Ubiquitin is the founding member of the UBL family. It participates in many biological processes, but its most studied effect is its covalent conjugation with other proteins, which targets proteins modified for proteasome-mediated degradation. Ubiquitin is one of the most conserved proteins in eukaryotes, however, it is found neither in bacteria nor in archaea. In humans, 14 copies of ubiquitin are expressed from different loci. Ubiquitin translates as an inactive precursor that is activated by means of a proteolytic cleavage immediately behind its amino acid glycine 76 by the same enzymes also responsible for the deubiquitination of ubiquitin substrates. Mature and active ubiquitin is a small globular protein of 76 amino acids (~8 KDa) [107]. Covalent modification of proteins by ubiquitin (ubiquitination) is defined as the formation of an isopeptide bond between the ε-amino group of a lysine in the target protein and the C-terminal glycine (G76) of ubiquitin. Ubiquitination occurs in different ways that regulate biological processes. It changes the specific properties of the target proteins that depend on i) the target itself and ii) the way ubiquitin binds. Ubiquitination of proteins with a single ubiquitin molecule (mono-ubiquitination) has been described as regulating biological processes such as endocytosis, trafficking, and gene expression regulation.

To exemplify the method of the invention VHH against ubiquitin were developed.

### Example 2. The simple density gradient method of the invention and its use to obtain nanoantibodies against ubiquitin

First, the inventors obtained the various chains of polyubiquitins (K6, K11, K27, K29, K33, K48 and K63). An alpaca named Nick was immunized four times with 100 µg of the K48 polyubiquitin chain. Then, after the third immunization, a significant increase in IgG antibodies was observed in alpaca serum qualitatively rapidly by Dot Blot analysis, by immobilization of the epitope in a nitrocellulose membrane and by the use of alpaca serum as a source of primary antibodies. Therefore, the inventors quickly built a bacterial presentation library consisting of 1 × 10⁶ clones of individual nanoantibodies with 1.2% vector religation.

### Building immunization and HHV libraries

The alpaca immunization process followed the protocol "Animal use in research" generated by the Bioethics Committee of the Universidad Austral de Chile. One day before immunization, 5 ml of blood was collected for preimmune serum testing. For immunization (day 1), 100 µg of polyubiquitin k48 chain was used. The cold lyophilized protein was dissolved in 2 ml of adjuvant (veterinary vaccine adjuvant, GERBU FAMA) diluted 1:1 in sterile water and injected subcutaneously into a male alpaca (*Vicugna pacos*)*.* A total volume of 4 ml was injected into four different sites of the alpaca. A 5 ml blood sample was collected seven days after the first immunization. On day 14, the alpaca was immunized again with 100 µg of the polyubiquitin k48 chain and so on for two more immunizations, until a day after the fourth immunization a 120 ml sample of blood was collected from the jugular vein in tubes containing 3.8% sodium citrate as an anticoagulant. And a library was generated in the same way as indicated for example 1.

Once the library was obtained, the inventors applied the method of the invention for the selection of nanoantibodies based on a simple density gradient.

### Coupling epitopes to beads

1 ml of NHS-activated sepharose 4 Fast Flow beads (General Electric) with 2 ml of cold HCl 1 mM were washed immediately before use, then washed 5 times with cold sterile PBS. 200 µg of polyubiquitin k48 chains in PBS1X were added to the beads and incubated in rotation until the next day. The groups that did not react in the medium were blocked by the addition of ethanolamine at a final concentration of 0.5 M. The beads were washed 5 times with PBS 1X and stored at 4°C.

### Density gradient separation

1 ml of glycerol stock solution from the library was inoculated into a flask containing 20 ml of LB medium with 25 µg/ml chloramphenicol and 2% glucose. The flask was incubated (pre-inoculum) until the next day at 37 °C with 200 rpm stirring. The same procedure was repeated with control bacteria transformed with a kanamycin-resistant plasmid (control). The preinoculum was sedimented and resuspended in LB medium with 25 µg mL-1 chloramphenicol and then diluted to 0.02 OD 600 nm in 100 ml of fresh LB medium with 25 µg mL-1 chloramphenicol without glucose, incubated at 37 °C with 200 rpm stirring to a OD 600 nm of 0.45 to 0.6. IPTG was added at a final concentration of 50 µM to induce protein expression for 3 hours at 30 °C and 200 rpm. The OD 600 nm absorbance of the library and control bacteria cultures was measured. 50 mL of both cultures were washed three times with 10 mL of filtered PBS 1C. Centrifugation was always at 3000 x g for 5 min. Both cultures were resuspended in a final volume of 10 ml of PBS1X. 2 ml of library culture and 2 ml of control culture were mixed (if the final 600 nm optical density was the same, if not, the volume of the control bacteria was adjusted according to the optical density to ensure an equal number of bacteria) and incubated with 300 µL of NHS beads coupled to the epitope protein in a 15 ml conical tube on a rotating platform for 30 min at room temperature. The mixture was added to Ficoll 6 ml (Ficoll-PaqueTM PLUS GE Healthcare) in a 15 ml conical tube and centrifuged at 200xg for 1 min. Unbound fraction was discarded (upper fractions)

The visible bead sediment contains bacteria that express an HHV that binds to antigens, in this case ubiquitin. This sediment was resuspended in 4 ml of PBS1X and rotated for 5 min at room temperature. This step was repeated six times, in order to eliminate any bacteria not attached to the beads.

In order to amplify the bacteria attached to the beads, 1 mL of LB medium was added and incubated for 5 min at room temperature, then 50 µL were seeded in LB agar plates with 25 µg/mL chloramphenicol and 2% glucose incubated at 37 °C until the next day (> 20 hours recommended).

Where each colony obtained corresponds to a bacterium containing a nanoantibody or HHV that binds specifically to the antigen of interest

### Confirmation of antigen binding.

The bacterial presentation system expresses nanoantibodies on the surface of bacteria fused with an intein protein and a myc tag. Buffer conditions were optimized to extract nanoantibody-intein fusion from the bacterial membrane and bacterial extract was used directly to confirm binding to polyubiquitin chains by dot blot. After selection of nanoantibodies using our simple density gradient protocol, 100 colonies were used to inoculate liquid LB medium and additionally induced for the expression of intein nanoantibodies. The cells were lysed under optimized conditions and the extract was used as a source of nanoantibodies as primary antibodies for the selection of antibodies that bind effectively by dot blot of the various chains of polyubiquitins immobilized in a nitrocellulose membrane.

They were revealed by sequential incubation with mouse anti-myc antibody and an antibody conjugated with anti-mouse HRP.

In this way, it was possible to identify Nb. No. 34 capable of recognizing all the ubiquitin chains tested by Dot Blot **(****Figure 3****)** and also by Western Blot **(****Figure 4****).** This was expressed in the plasmid pNae2 (fused with intimin) with 6×His and Myc tags and then purified by nickel affinity chromatography. To perform the western blot, the different chains of polyubiquitins were separated electrophoretically in a 12% polyacrylamide gel and then transferred by electrotransfer to a nitrocellulose membrane which was incubated with the extract of Nb. No. 34 and successively with anti-myc antibody and anti-mouse antibody coupled to HRP.

Thus, the invention proved useful for quickly and efficiently selecting nanoantibodies that bind specifically to an antigen of interest.

## Claims

**1.** Method of separation of nanoantibodies (HHV) against a specific antigen from a nanoantibody expression library **CHARACTERIZED in that** it comprises the following steps:
(a) binding the antigen of interest to beads made of protein-binding polymers
(b) incubating the microorganisms of the expression library with the beads of step (a), where the expression library corresponds to microorganisms transformed with the cDNA of fragments corresponding to the HHV domains of HHV-producing animal previously immunized with that antigen of interest, or synthetic libraries;
(c) placing the beads of step (b) in a tube with an inert medium with a density greater than or equal to 1 g/mL and centrifuge for 45 s to 2 minutes at a rate between 150 and 250 g;
d) discarding the upper fraction and supernatant with the free microorganisms, and obtaining the beads with the linked microorganisms, which correspond to those that express HHV that recognizes the antigen.

**2.** Method according to claim 1 **CHARACTERIZED in that** in step (a) the beads are sepharose, cellulose, latex, agarose or nickel and are modified to bind proteins.

**3.** Method according to claim 2 **CHARACTERIZED in that** in the beads have a density greater than the medium used in (c).

**3.** Method according to claim 1 **CHARACTERIZED in that** in step (c) the inert medium is reactive Ficol, percol or sucrose.

**4.** Method according to claim 3 **CHARACTERIZED in that** in step (a) the beads or equivalents bind stably to the antigen of interest in a time of between 2 to 12 hours.

**5.** Method according to claim 4 **CHARACTERIZED in that** sites which have not reacted with the antigen are blocked.

**6.** Method according to claim 1 **CHARACTERIZED in that** the microorganisms of the expression library are treated with a protein expression inducer for 2 to 4 hours prior to step (b).

**7.** Method according to claim 6 **CHARACTERIZED in that** the protein expression inducer is Isopropyl-β-D-1-tiogalactopyranoside (IPTG) at a concentration between 20 to 100 µM.

**8.** Method according to claim 1 **CHARACTERIZED in that** in step (b) is incubated for between 20 to 60 minutes at room temperature.

**9.** Method according to claim 1 **CHARACTERIZED in that** in step (d) the beads are washed with PBS and seeded on agar plates with culture medium in order to obtain isolated colonies.

**10.** Method according to claim 9 **CHARACTERIZED in that** each colony corresponds to a microorganism expressing an HHV capable of binding to the antigen of interest.
